# EUROPEAN PATENT SPECIFICATION

(11) **EP 0 968 166 B1**
(45) Date of publication and mention of the grant of the patent: **12.06.2002**
(21) Application number: 98909484.2
(22) Date of filing: 24.02.1998
(51) Int. Cl.: C07C 51/14, C07C 51/12, C07C 53/128

(54) **PROCESS FOR THE MANUFACTURE OF CARBOXYLIC ACIDS**
VERFAHREN ZUR HERSTELLUNG VON CARBONSÄUREN
PROCEDE POUR LA FABRICATION D'ACIDES CARBOXYLIQUES

(30) Priority: 25.02.1997 EP 97200537
(43) Date of publication of application: 05.01.2000
(73) Proprietor: Resolution Research Nederland B.V., 1031 CM Amsterdam (NL)
(72) Inventor: BREED, Anthonius, Johannes, Maria, NL-1031 CM Amsterdam (NL); HAAN, Rene, Johan, NL-1031 CM Amsterdam (NL); LANGE, Jean-Paul, NL-1031 CM Amsterdam (NL); PETRUS, Leonardus, NL-3196 KK Rotterdam (NL)
(74) Representative: Wittop Koning, Tom Hugo
(86) International application number: EP9801128
(87) International publication number: WO9838149

(56) References cited:
- EP-A- 0 249 976
- WO-A-96/20154
- US-A- 5 250 726

## Description

The invention relates to a process for the manufacture of carboxylic acids. More in particular the invention relates to a process for the manufacture of branched carboxylic acids by means of a Koch synthesis using carbon monoxide as reagent and a solid acid catalyst.

The up to now available processes are characterized by the fact that no solid acid catalyst could be used, unless said catalyst is operated under unattractively severe conditions or unless said catalyst is combined with corrosive Lewis acid cocatalyst or unless said catalyst is used in a non-aqueous reaction system.

In particular from International Application WO 96/20154 was known a process for the production of trialkylacetic acids from branched olefins and carbon monoxide in a non-aqueous reaction system using a solid resin catalyst comprising a cationic resin, having sufficient acid groups to provide requisite protons for conversion of branched olefin and carbon monoxide to trialkylacetic acids.

In particular the cationic resin was specified to have an acidity of at least equivalent to that of a 65 wt% sulphuric acid.

It will be appreciated by an average person skilled in the art that the said process can only be performed in two steps, in the first step of which stoichiometric amounts of branched olefin and water will not lead to the desired products in an acceptable yield. Moreover, said process cannot produce more than 1 mole of converted olefin per mole active proton on the solid catalyst in one cycle of two steps.

On the other hand from WO 92/18592 was known a process for the manufacture of trialkylacetic acids and particularly of pivalic acid, from branched olefins and particularly isobutene, and carbon monoxide, using a solid acid catalyst together with minor amounts of a Lewis acid, such as boron trifluoride.

In addition from EP-A-0249976 was known a process for the manufacture of branched carboxylic acids, by catalytic conversion of olefins with carbon monoxide and water in the presence of zeoliths as catalysts at temperatures of from 200 to 500 °C and at pressures of 200 to 700 bar.

More in particular zeoliths of the pentasil type are used as catalysts. According to the exemplified embodiments only high temperatures (300 °C) and pressures (300-500 bar) are used.

It will be appreciated that said disclosed reaction conditions will give rise to higher operation costs due to required measures as to safety and environment.

Therefore there is still a strong need for further improvement of the manufacturing process of branched carboxylic acids, starting from branched olefins and carbon monoxide.

An object of the present invention is providing an improved manufacturing process for branched carbox ylic acids, which process uses relatively mild conditions on the one hand and which shows high conversion and high selectivity to branched acids on the other hand. As a result of extensive research and experimentation such an improved process aimed at has been surprisingly found.

Accordingly the invention relates to a process for manufacture of branched carboxylic acids from branched olefins by means of reaction with carbon monoxide and a solid acid catalyst, characterized in that a branched olefin, or a precursor thereof, is reacted in continuous backmixed reactor, wherein sufficient stirring of the feed components and product occurs to obtain an efficient backmixing with continuously supplied carbon monoxide and water, while continuously an effluent is withdrawn comprising branched carboxylic acid, non-converted olefin, carbon monoxide and water, in the presence of an acidic ion exchanger, having sufficient acid groups to provide requisite protons for conversion of said olefin or a precursor of it, and carbon monoxide into branched carboxylic acids, and at a temperature in the range of from 25 to 200 °C and at a pressure in the range of from 10 to 200 bar, while the water/olefin ratio is in the range of from 0.5 to 2 mole/mole.

More in particular the invention relates to an improved manufacturing process of trialkylacetic acids of the formula 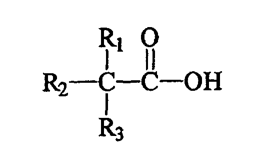 wherein each symbol R represents a radical having 1 to 10 carbon atoms.

More preferably the total number of carbon atoms in the trialkylacetic acids ranges from 5 to 19 and most preferably from 5 to 14, derived from C4-C14 olefins.

With the term "branched olefin or a precursor thereof" as used throughout the present specification is meant that branched olefin itself as well as alcohols, esters or ethers, from which the specific olefin can be easily derived, can be used as starting materials for the present manufacturing process, which makes this process much more flexible than conventional prior art processes.

In general all olefins containing at least one tertiary carbon atom or precursors therefor, can be converted by the present process.

An important advantage of the process of the present invention is that it shows an improved combination of high conversion degree and high selectivity as to the desired branched carboxylic acid, in comparison to these conventional prior art processes, while operated at relatively mild conditions.

The catalyst to be used for the process of the present invention is a solid acidic ion exchanger showing strong acid behaviour selected from the group consisting of sulfonated resins, sulfonated poly(tetrafluoro-ethylene) and sulfonated siloxane polymers. Preferably, the solid acidic ion exchanger is selected from sulfonated copolymers of styrene and divinylbenzene, phenol resins or phenolic based resins.

In either case of the presence of active sulfonic acid groups, the resin is treated to give a sulfonic acid cation-exchange resin capable of providing sufficient protons, i.e. the resin having per active site an acid strength equivalent to at least 65 wt% sulphuric acid and preferably to at least 70 wt% sulphuric acid.

More preferred catalysts are sulfonated copolymers derived from styrene and divinylbenzene and having a sulfon group density of > 2 meq/ml dry resin and preferably >3 meq/ml dry resin, while the copolymer has a content of divinylbenzene in the range of 4 to 30 wt% and preferably 8 to 18 wt%.

Specific more preferred examples of commercial effective catalysts are AMBERLYST 15, NAFION or DELOXAN catalysts (AMBERLYST, NAFION and DELOXAN are Trade Marks).

Most preferred are the AMBERLYST or NAFION type catalysts. The reaction temperature in the CSTR is preferably in the range of from 100 to 150 °C.

The pressure in the reactor is preferably in the range of from 50 to 100 bar.

During the reaction an inert organic solvent can be used, which does not interfere with the desired reaction, preferably a solvent which can easily be separated from the other reaction mixture components and recirculated. As organic solvents can be used apolar as well as polar solvents such as ketones, ethers, substituted aromatics, esters and carboxylic acids.

According to a more preferred embodiment of the present process, the branched acid primarily to be produced, is present as solvent in the reactor, and is regularly discharged from it together with water, CO, non-converted olefin and by-products, to keep the liquid level in the reactor constant.

Normally the CSTR is filled with solvent and catalyst with a catalyst/solvent wt ratio of in the range of from 0.1 to 0.5 w/w and preferably 0.2-0.3 w/w. The respective reactants are continuously introduced into the reactor and reaction mixture is continuously discharged.

The feed of starting olefin is in the range of from 0.01 to 10 g/g/hr, while the water/olefin molar ratio is in the range of from 0.5 to 2 mole/mole and preferably about 1 and the CO/olefin molar ratio is in the range of from 0.5 to 1000 mole/mole and preferably from 1 to 100.

It will be appreciated that, when using water amounts significantly below the hereinbefore specified amounts, the process becomes unattractive due to too low selectivity and that the selectivity and conversion have surprisingly been improved when using stoichiometric water:olefin = 1:1 feed.

The invention is further illustrated by the following examples, however without restricting its scope to these specific embodiments.

### EXAMPLE 1

56 g of dried Amberlyst 15 were loaded in a 300 ml CSTR reactor, suspended in 145 ml of n-hexanoic acid (solvent), and activated upon heating up to 155 °C under 10 bar CO with regular purge of the gas cap followed by 1 h at 155 °C under a 80 bar CO flow of 50 g/h. A feed containing propylene trimer water and CO the molar ratio of water:trimer being 1:1 was then admitted to the reactor with a velocity of 8.5, 1.2 and 50 g/h (WHSV of 0.15, 0.021 and 0.9 g/g/h) under continuous stirring of 1100 rpm, the liquid level of the reactor being kept constant by continuously removing the excess liquid product.

Under these conditions the reaction proceeded with about 75-85% conversion and 93-95% selectivity to the V10 branched carboxylic acid having 10 carbon atoms, for some 24 h.

### COMPARATIVE EXAMPLE 1

20 g of Amberlyst 15 were loaded in a batch rector, dried for 2 h at 110 °C under vacuum, suspended in a solution of 50 g propanoic acid (solvent), 6 g PT3 and 0.8 g water and, finally, heated to 150 °C under 80 bar CO for 65 h.

Under these conditions the reaction proceeded with - about 91% conversion and 8% selectivity to the branched carboxylic acid having 10 carbon atoms.

### EXAMPLE 2

In the same way as described in example 1 NAFION NR 50 catalyst (59 g), dissolved in 120 ml hexanoic acid was used for the conversion of propylene trimer with CO and H2O into branched carboxylic acids, containing 10 carbon atoms, under the following conditions

| | |
|---|---|
| propylene trimer | 8.8 g/h (WHSV = 0.16 g/g/h) |
| water | 1.2 g/h (WHSV = 0.021 g/g/h) |
| CO | 52 g/h (WHSV = 0.9 g/g/h) |
| temperature | 155 °C |
| pressure | 80 bar |

The conversion was 88 mol% and the selectivity was 91 mol%.

## Claims

1. A process for manufacture of branched carboxylic acids from branched olefins by means of reaction with carbon monoxide and a solid acid catalyst, **characterized in that** a branched olefin, or a precursor thereof, is reacted in continuous backmixed reactor, wherein sufficient stirring of the feed components and product occurs to obtain an efficient backmixing with continuously supplied carbon monoxide and water, while continuously an effluent is withdrawn comprising branched carboxylic acid, non-converted olefin, carbon monoxide and water, in the presence of an acidic ion exchanger, having sufficient acid groups to provide requisite protons for conversion of said olefin or a precursor of it, and carbon monoxide into branched carboxylic acids, and at a temperature in the range of from 25 to 200 °C and at a pressure in the range of from 10 to 200 bar, while the water/olefin ratio is in the range of from 0.5 to 2 mole/mole.

2. Process according to claim 1, **characterized in that** it is carried out at a pressure in the range of from 50 to 100 bar.

3. Process according to claims 1 and 2, **characterized in that** it is carried out at a temperature in the range of from 100 to 150 °C.

4. A Process according to claims 1-3, **characterized in that** trialkylacetic acids of the formula 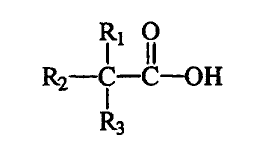 are produced, wherein each symbol R represents a radical having 1 to 10 carbon atoms.

5. A process according to claims 1-4, **characterized in that** the total number of carbon atoms in the trialkylacetic acids ranges from 5 to 14.

6. A process according to claims 1-5, **characterized in that** as solid acid catalyst is used a solid acidic ion exchanger, selected from the group consisting of sulfonated resins, sulfonated poly(tetrafluoro-ethylene) and sulfonated siloxane polymers.

7. A process according to claims 1-6, **characterized in that** as catalyst is used an acidic ion exchanger selected from sulfonated copolymers of styrene and divinylbenzene, phenol resins or phenolic base resins.

8. A process according to claims 6-7, **characterized in that** the resin is treated to give a sulfonic acid cation-exchange resin, such that the resin having per active site an acid strength equivalent to at least 65 wt% sulphuric acid and preferably to at least 70 wt% sulphuric acid.

9. A process according to claims 1-8, **characterized in that** as catalyst a sulfonated copolymer derived from styrene and divinylbenzene, having a sulfon group density of >2 meq/ml dry resin and preferably >3 meq/ml dry resin, while the copolymer has a content of divinylbenzene in the range of 4 to 30 wt% and preferably from 8 to 18 wt%.

10. A process according to claims 1-9, **characterized in that** during the reaction a branched acid primarily to be produced, is present as solvent in the reactor.

## Patentansprüche

1. Verfahren zur Herstellung von vernetzten Carbonsäuren aus vernetzten Olefinen mittels einer Reaktion mit Carbonmonoxid und einem festen Säurekatalysator, **dadurch gekennzeichnet, dass** ein vernetztes Olefin oder ein Vorprodukt davon in einem kontinuierlichen Reaktor mit Rückmischung zur Reaktion gebracht wird, in welchem die zugeführten Stoffe und das Produkt ausreichend verrührt werden, um eine wirksame Rückmischung mit kontinuierlich zugeführtem Carbonmonoxid und Wasser zu erreichen, wobei kontinuierlich in Anwesenheit eines sauren Ionentauschers ein Ausfluss abgezogen wird, der vernetzte Carbonsäure, nicht umgewandeltes Olefin, Carbonmonoxid und Wasser enthält und wobei der Ionentauscher ausreichend Säuregruppen aufweist, um die erforderlichen Protonen zur Umwandlung des genannten Olefins oder eines Vorproduktes davon zur Verfügung zu stellen und um Carbonmonoxid in vernetzte Carbonsäuren umzuwandeln, wobei die Temperatur in der Größenordnung von 25 bis 200 °C und der Druck in der Größenordnung von 10 bis 200 bar und das Verhältnis Wasser/Olefin in der Größenordnung von 0,5 bis 2 mol/mol liegt.

2. Verfahren nach Anspruch 1, **dadurch gekennzeichnet, dass** es bei einem Druck in der Größenordnung von 50 bis 100 bar ausgeführt wird.

3. Verfahren nach den Ansprüchen 1 und 2, **dadurch gekennzeichnet, dass** es bei einer Temperatur in der Größenordnung von 100 bis 150 °C ausgeführt wird.

4. Verfahren nach den Ansprüchen 1 - 3, **dadurch gekennzeichnet, dass** Trialkyl-Essigsäuren der Formel 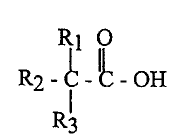 hergestellt werden, wobei jedes Symbol R ein Radikal mit 1 bis 10 Kohlenstoffatomen darstellt.

5. Verfahren nach den Ansprüchen 1 bis 4, **dadurch gekennzeichnet, dass** die Gesamtzahl der Kohlenstoffatome in den Trialkyl-Essigsäuren zwischen 5 und 14 liegt.

6. Verfahren nach den Ansprüchen 1 bis 5, **dadurch gekennzeichnet, dass** als fester Säurekatalysator ein fester, saurer Ionentauscher benutzt wird, der aus der Gruppe ausgewählt wurde, die aus sulfonierten Harzen, sulfonierten Poly(tetrafluorethylen) und sulfonierten Siloxan-Polymeren besteht.

7. Verfahren nach den Ansprüchen 1 bis 6, **dadurch gekennzeichnet, dass** als Katalysator ein saurer Ionentauscher benutzt wird, der aus sulfonierten Copolymeren aus Styrol und Divinylbenzol, Phenolharzen oder Harzen auf Phenolbasis ausgewählt wurde.

8. Verfahren nach den Ansprüchen 6 bis 7, **dadurch gekennzeichnet, dass** das Harz derart behandelt wird, dass ein Sulfonsäure-Kationenaustauschharz entsteht, so dass das Harz pro aktiver Stelle eine Säurestärke von zumindest 65 Gew.-% von Schwefelsäure und vorzugsweise zumindest 70 Gew.-% von Schwefelsäure hat.

9. Verfahren nach den Ansprüchen 1 bis 8, **dadurch gekennzeichnet, dass** als Katalysator ein sulfoniertes Copolymer verwendet wird, welches aus Styrol und Divinylbenzol abgeleitet ist und eine Sulfongruppendichte von >2 Milliäquivalent/ml trockenem Harz und vorzugsweise >3 Milliäquivalent/ml trockenem Harz hat, während das Copolymer einen Gehalt von Divinylbenzol in der Größenordnung von 4 bis 30 Gew.-% und vorzugsweise von 8 bis 18 Gew.-% hat.

10. Verfahren nach den Ansprüchen 1 bis 9, **dadurch gekennzeichnet, dass** während der Reaktion eine vernetzte Säure, welche primär zu erzeugen ist, als Lösungsmittel in dem Reaktor anwesend ist.

## Revendications

1. Un procédé pour la fabrication d'acides carboxyliques ramifiés à partir d'oléfines ramifiées, par le biais d'une réaction avec le monoxyde de carbone et un catalyseur acide solide, **caractérisé par le fait qu'**une oléfine ramifiée, ou un précurseur de celle-ci, est mis à réagir dans un réacteur continu à rétromélange dans lequel un brassage suffisant des composés alimentés et du produit s'opère pour obtenir un mélange en retour efficace avec du monoxyde de carbone et de l'eau apportés en permanence, tandis qu'un effluent est soutiré en continu, effluent contenant un acide carboxylique ramifié, l'oléfine non transformée, du monoxyde de carbone et de l'eau, en présence d'un échangeur d'ions acide qui possède suffisamment de groupes acides pour apporter les protons nécessaires à la transformation de la dite oléfine ou d'un précurseur de celle-ci et du monoxyde de carbone en acides carboxyliques ramifiés, à une température comprise entre 25 et 200°C, et à une pression comprise entre 10 et 200 bar, alors que le rapport eau/oléfine se situe entre 0,5 et 2 mole/mole.

2. Un procédé suivant la revendication 1, **caractérisé par le fait qu'**il est réalisé à une pression comprise entre 50 et 100 bar.

3. Un procédé suivant les revendications 1 et 2, **caractérisé par le fait qu'**il est réalisé à une température comprise entre 100 et 150°C.

4. Un procédé suivant les revendications 1 à 3, **caractérisé par le fait que** des acides trialkylacétiques de formule 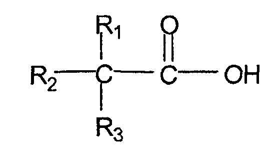 sont produits, dans lesquels chaque symbole R représente un radical possédant 1 à 10 atomes de carbone.

5. Un procédé suivant les revendications 1 à 4, **caractérisé par le fait que** le nombre total d'atomes de carbone dans les acides trialkylacétiques varie entre 5 et 14.

6. Un procédé suivant les revendications 1 à 5, **caractérisé par le fait qu'**un échangeur d'ions acide solide, choisi dans le groupe composé des résines sulfonées, du poly(tétrafluoroéthylène) sulfoné et des polymères de siloxane sulfonés, est utilisé comme catalyseur acide solide.

7. Un procédé suivant les revendications 1 à 6, **caractérisé par le fait qu'**un échangeur d'ions acide, choisi parmi des copolymères sulfonés de styrène et de divinylbenzène, des résines phénoliques ou des résines à base phénolique, est utilisé comme catalyseur.

8. Un procédé suivant les revendications 6 et 7, **caractérisé par le fait que** la résine est traitée pour donner une résine échangeuse de cations d'acide sulfonique, de telle sorte que la résine possède par site actif une acidité équivalant à au moins 65% en poids d'acide sulfurique, et de préférence à au moins 70% en poids d'acide sulfurique.

9. Un procédé suivant les revendications 1 à 8, **caractérisé par le fait qu'**un copolymère sulfoné dérivé du styrène et du divinylbenzène, possédant une densité de groupes sulfonés supérieure à 2 mEq/ml de résine sèche et de préférence supérieure à 3 mEq/ml de résine sèche, et une teneur en divinylbenzène variant de 4 à 30% en poids et de préférence comprise entre 8 et 18% en poids, est utilisé comme catalyseur.

10. Un procédé suivant les revendications 1 à 9, **caractérisé par le fait qu'**au cours de la réaction, un acide ramifié à produire en premier lieu est présent en tant que solvant dans le réacteur.
